# FASCICULE DE BREVET EUROPEEN

(11) **EP 3 268 008 B1**
(45) Date de publication et mention de la délivrance du brevet: **12.02.2020**
(21) Numéro de dépôt: 16714537.4
(22) Date de dépôt: 11.03.2016
(51) Int. Cl.: A61K 31/575, A61K 31/58, A61K 45/06, C07J 41/00, C07J 43/00, A61P 31/00, A61P 31/04, A61P 31/10, A61P 31/12, A61P 33/00, A61P 35/00

(54) **COMPOSÉS ANALOGUES DE LA SQUALAMINE UTILES COMME AGENTS ANTIBACTÉRIENS**
VERBINDUNGEN, DIE ANALOGA VON SQUALAMIN SIND, MIT VERWENDUNG ALS ANTIBAKTERIELLE MITTEL
COMPOUNDS THAT ARE ANALOGS OF SQUALAMINE, USED AS ANTIBACTERIAL AGENTS

(30) Priorité: 12.03.2015 FR 1552048
(43) Date de publication de la demande: 17.01.2018
(73) Titulaire: VIRBAC, 06516 Carros (FR)
(72) Inventeur: BRUNEL, Jean-Michel, 13012 Marseille (FR); MARC, Jean-Pascal, 06570 Saint Paul De Vence (FR)
(74) Mandataire: Gevers & Orès
(86) Numéro de dépôt international: PCT/IB2016/051408
(87) Numéro de publication internationale: WO 2016/142922

(56) Documents cités:
- WO-A1-2011/067501
- WO-A1-2013/104849
- WO-A2-96/40728
- WO-A2-2009/032321
- BRIAN T. WALKER ET AL: "Squalamine and its derivatives as potential antitubercular compounds", TUBERCULOSIS, vol. 93, no. 1, 1 janvier 2013 (2013-01-01), pages 102-103, XP055223342, GB ISSN: 1472-9792, DOI: 10.1016/j.tube.2012.08.002

## Description

L'invention concerne des analogues de la squalamine pour leur utilisation dans le traitement des infections bactériennes, ou fongiques, chez l'homme ou l'animal, ainsi que les compositions pharmaceutiques ou vétérinaires les comprenant.

En 1993, la squalamine, un stéroïde naturel, isolée majoritairement des tissus d'un petit requin *Squalus acanthias,* s'est révélée être une substance très active présentant essentiellement une activité antiangiogénique contre les cellules et une activité anti virale et antibactérienne.

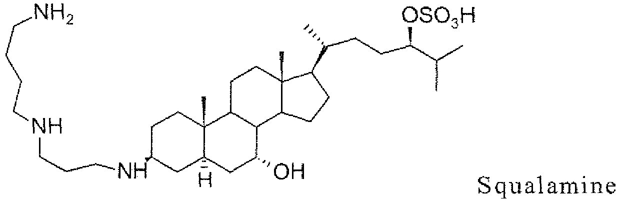

Chimiquement, la squalamine est une molécule originale présentant un caractère amphiphile. Elle comporte ainsi, une partie centrale apolaire (un squelette de type cholestane) et deux extrémités polaires (une chaîne polyamine et un groupement sulfate).

Initialement, ce polyaminostérol hydrosoluble avait suscité l'intérêt pour ses propriétés antiangiogéniques et antimicrobiennes sur une variété de bactéries à Gram positif (*Staphylococcus aureus, Enterococcus faecalis*) et à Gram négatif (*Escherichia coli, Pseudomonas aeruginosa*), des champignons (*Candia albicans, Candida tropicalis*) et des protozoaires.

La source naturelle de la squalamine étant limitée, on a recherché des dérivés synthétiques analogues aminostéroïdiens de la squalamine. On a décrit notamment des dérivés ou analogues comportant une chaîne polyaminée en position 3 ou 7 de cycles 10, 13 diméthyl, 17 octane cholestane ou cholestène, éventuellement hydroxylés en position 7 ou respectivement 3. En particulier, des dérivés de formule IIa - IIb - IIc - IId et II-1 ci- après on été décrits comme présentant une activité antibactérienne similaire à la squalamine vis-à-vis de diverses bactéries Gram positif et Gram négatif multirésistantes (WO 2011/067501 et références 1 à 7).

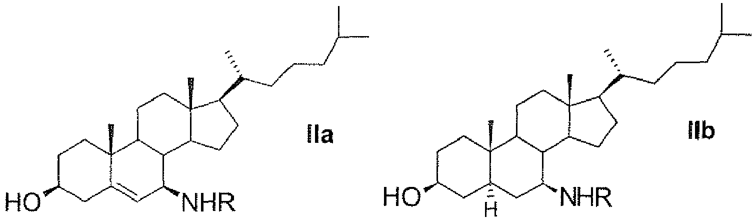

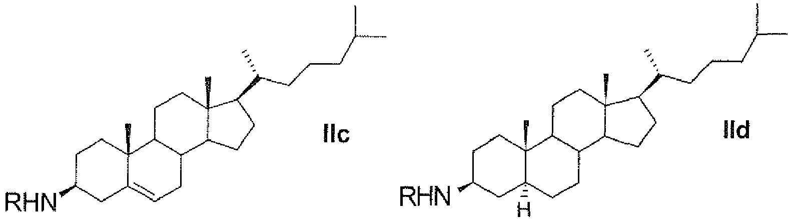

On a plus particulièrement suggéré une application de ces dérivés pour un traitement curatif des infections pulmonaires par voie aérosol. Cependant, la Demanderesse a observé que ces composés présentaient une cytotoxicité importante et que les composés de formule IIc et IId présentaient une faible activité contre certaines bactéries gram-négatives telles que *E. coli.*

D'autres aminosterols ont été décrits comme présentant une activité contre les infections bactériennes ou fongiques dans WO 96/40728.

Il a maintenant été découvert des composés analogues de la squalamine, présentant une bonne activité antibactérienne contre des bactéries gram-positives et gram-négatives, tout en étant avantageusement moins cytotoxiques que la squalamine.

Ainsi, selon un premier objet, l'invention concerne un composé de formule (I) : 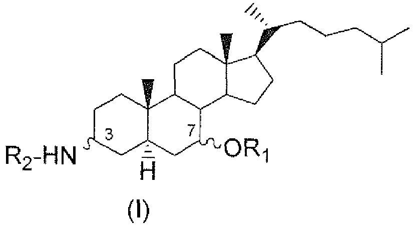 dans laquelle :
R₁ est choisi parmi H, SO₃H, un groupe alkyle en C₁-C₈, un groupe aryle en C₆-C₁₀, un groupe C(=O)R₁₁,
R₂ est tel que défini dans la revendication 1,
R₁₁ est choisi parmi un groupe alkyle en C₁-C₈, un groupe aryle en C₆-C₁₀,
ainsi que les stéréoisomères, mélanges de stéréoisomères, et/ou sels pharmaceutiquement acceptables de celui-ci, pour leur utilisation dans le traitement des infections bactériennes, ou fongiques, chez l'homme ou l'animal.

L'invention concerne également un composé de formule (I) : 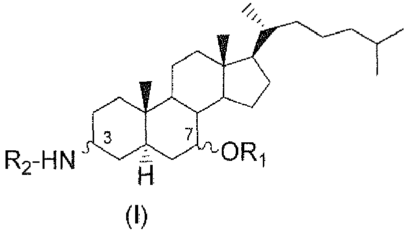 dans laquelle :
R₁ est choisi parmi H, SO₃H, un groupe alkyle en C¡-C₈, un groupe aryle en C₆-C₁₀, un groupe C(=O)R₁₁,
R₂ est tel que défini dans la revendication 1,
R₁₁ est choisi parmi un groupe alkyle en C₁-C₈, un groupe aryle en C₆-C₁₀,
ainsi que les stéréoisomères, mélanges de stéréoisomères, et/ou sels pharmaceutiquement acceptables de celui-ci, pour leur utilisation dans le traitement des infections bactériennes, ou fongiques, chez l'homme ou l'animal.

Le composé 3β-(norspermine)-7α-hydroxy-5α-cholestane et les composés de formule suivante sont exclus :

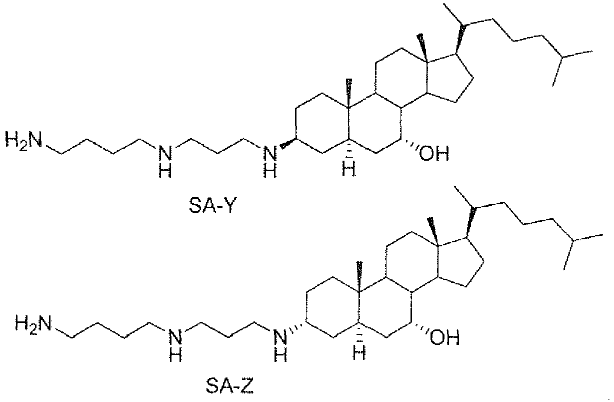

De préférence, R₁ est H.

L'invention concerne des composés de formule (I) dans laquelle le groupe -NHR₂ est choisi parmi :

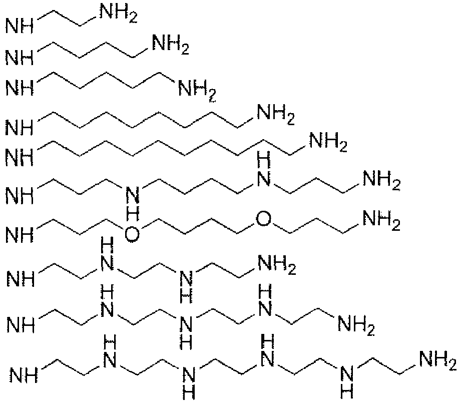

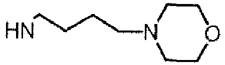

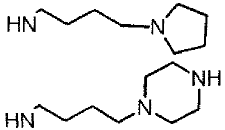

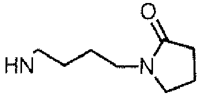

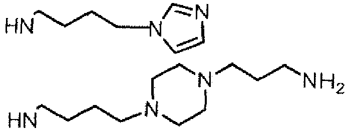

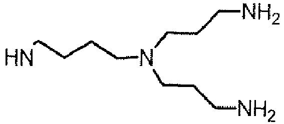

L'invention concerne des composés de formule (I) dans laquelle le groupe -NHR₂ est choisi parmi :

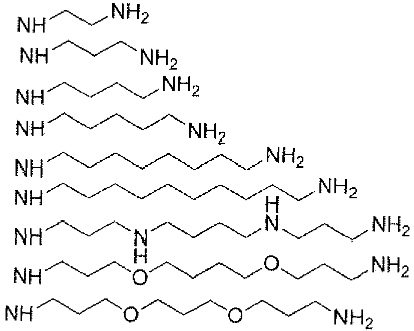

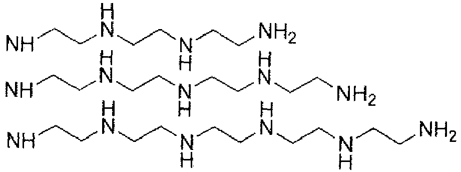

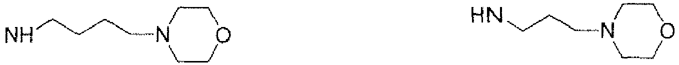

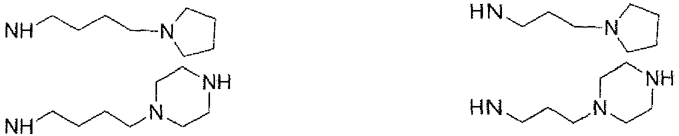

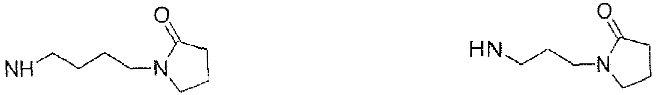

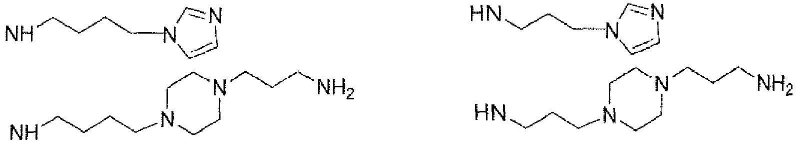

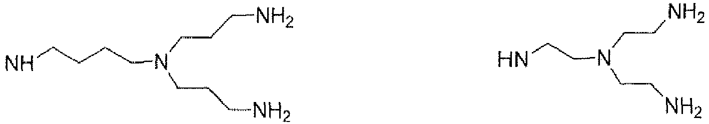

Selon une variante préférée, les composés de formule (I) sont choisis parmi :
- 3β-spermino-7α-hydroxy-5α-cholestane (SA-1)
- 3β-spermino-7β-hydroxy-5α-cholestane (SA-2)
- 3β-(1,3-diaminopropane)-7β-hydroxy-5α-cholestane (SA-4)
- 3β-(1,4-diaminobutane)-7β-hydroxy-5α-cholestane (SA-5)
- 3β-(Tris(2aminoethyl)amine)-7β-hydroxy-5α-cholestane (SA-6)
- 3β-(1,5-diaminopentane)-7α-hydroxy-5α-cholestane (SA-7)
- 3β-(1,4-bis(3-aminopropyl)piperazine)-7β-hydroxy-5α-cholestane (SA-8)
- 3β-(1,4-bis-(3-aminopropoxy)butane)-7β-hydroxy-5α-cholestane (SA-9)
- 3β-(1-(3-aminopropyl)imidazole)-7α-hydroxy-5α-cholestane (SA-11)
- 3β-(1-(3-aminopropyl)morpholine)-7α-hydroxy-5α-cholestane (SA-12)

Selon une autre variante préférée, les composés de formule (I) sont choisis parmi :
- 3β-spermino-7β-hydroxy-5α-cholestane (SA-2)
- 3β-(1,3-diaminopropane)-7β-hydroxy-5α-cholestane (SA-4)

Selon une autre variante préférée, l'invention inclut des composés de formule (I) pour le traitement d'infections bactériennes, notamment des infections bactériennes à Gram positif telles que des infections à *Staphylococcus aureus, Staphylococcus faecalis* et/ou à Gram négatif telles que *Escherichia Coli, Pseudonomas aeruginosa.*

Les composés de formule (I) selon l'invention sont notamment utiles pour le traitement antibiotique d'infections bactériennes, notamment de souches de bactéries Gram-positives ou Gram-négatives, chez l'homme ou l'animal. Selon un mode de réalisation, les composés de formules (I) sont utilisés chez l'animal, notamment chez le chien, le chat, ou les ruminants.

A titre d'exemple, les composés selon l'invention sont utiles pour le traitement de mammites, de métrites, d'infections dentaires, de pyodermites ou encore d'otites notamment chez l'animal. Les composés selon l'invention, sont également utiles pour la fabrication de produits destinés à détruire les biofilms.

Selon un mode de réalisation particulier, l'invention concerne le composé de formule (I) pour son utilisation dans le traitement de mammites, de métrites, d'infections dentaires, de pyodermites ou encore d'otites, notamment chez l'animal. Le composé selon l'invention est également utile pour la fabrication de produits destinés à détruire les biofilms.

La mammite ou mastite est l'inflammation de la mamelle chez les mammifères, c'est une infection courante en élevage des femelles laitières (vaches, brebis, chèvres, bufflonnes et chamelles). Elle est caractérisée par la présence dans le lait de cellules inflammatoires (leucocytes) et éventuellement de bactéries. Cette inflammation peut avoir des conséquences cliniques avec modification de l'aspect du lait, inflammation visible de la mamelle (tuméfaction, douleur, œdème) et éventuellement atteinte de l'état général. Le plus souvent la maladie demeure subclinique avec altération de la composition du lait et diminution de la production. La mammite résulte d'une infection de la mamelle par des bactéries plus ou moins adaptées à ce biotope. En élevage laitier spécialisé, les mammites provoquent des pertes économiques importantes (lait non produit, impropre à l'usage, altération de la qualité du lait) et constituent un risque de santé publique (bactéries pathogènes et résidus antibiotiques). Les mammites sont dues à la pénétration puis au développement d'une bactérie dans la glande mammaire. L'entrée du germe se fait généralement par l'extrémité du trayon. Une mammite ne concerne donc en général pas tous les quartiers du pis de l'animal. Les principales bactéries responsables de mammite peuvent être regroupées en deux ensembles, en fonction de leur réservoir de contamination : Les germes se trouvant à la surface de la mamelle : *Staphylocoques, Streptococcus agalactiae, Streptococcus disgalactiae, Streptococcus uberis.* Ces bactéries sont principalement responsables de mammites sub-cliniques (non détectables à l'œil nu) qu'il est parfois difficile de guérir en cours de lactation, la période de tarissement est alors mise à profit pour traiter les quartiers infectés aux antibiotiques. Les germes se trouvant dans l'environnement (litière): par exemple, *Streptococcus uberis, Escherichia coli.* Ces bactéries entrainent généralement des mammites cliniques, qui peuvent aller jusqu'à la mort rapide de l'animal en l'absence de traitement adapté. Les mammites à mycoplasmes posent encore des problèmes dans les cheptels caprins, même si elles ont actuellement quasiment disparues des troupeaux bovins.

La métrite est une inflammation de l'ensemble de la paroi utérine. Elle est causée par une infection bactérienne et elle est presque toujours observée après une mise bas anormale ou une infection utérine importante. Sa gravité s'échelonne d'une infection subclinique à une maladie déclarée avec fièvre et diminution de la production de lait. La métrite peut prédisposer les vaches à la cétose, au déplacement de la caillette et à d'autres troubles du post-partum. Elle peut également aboutir à une baisse de la fertilité, temporaire ou permanente, et même, dans certains cas, à la mort de l'animal. La métrite est souvent liée à une contamination de l'utérus par la bactérie *Arcanobacterium pyogenes,* soit seule soit conjointement à d'autres micro-organismes pathogènes tels que: *Fusobacterium necrophorum, Bacteroides spp.* ou *Escherichia coli*. Juste après le vêlage, l'utérus constitue un environnement idéal pour la croissance bactérienne. Durant la première semaine post-partum, jusqu'à 90% des vaches sont victimes d'une infection utérine d'origine bactérienne.

La pyodermite est une maladie cutanée purulente, qui peut être aiguë ou chronique, locale ou diffuse. La pyodermite est étymologiquement une infection de la peau. Elle est d'origine externe, causée par une bactérie, généralement le *staphylocoque* ou le *Streptococcus pyogenes.* Une pyodermite peut être circonscrite ou généralisée. Chez le chien, on observe souvent une dermatite pyotraumatique. Il s'agit d'une lésion cutanée résultant d'une compulsion à griffer, mordiller et lécher une partie du corps. Dès que la lésion est assez importante, une infection secondaire par des bactéries opportunistes peut survenir, amenant l'animal à mordiller ou à se griffer davantage. La plupart des animaux souvent affectés ont des allergies : particulièrement les animaux allergiques aux puces. Cependant, n'importe quelle irritation cutanée peut provoquer une dermatite pyotraumatique.

L'otite est une inflammation du conduit auditif. Il s'agit d'une pathologie extrêmement fréquente chez les carnivores domestiques, en particulier le chien. Elle peut avoir de nombreuses origines dont certaines seront responsables d'otites récidivantes. Plusieurs types de bactéries (*Staphylocoques, Pseudomonas...*) et de levures (*Malassezia*) peuvent se développer dans le conduit auditif, provoquant l'apparition d'une otite. Ces otites sont alors associées à des sécrétions purulentes et à une odeur très désagréable.

La maladie parodontale ou infection dentaire est la principale cause de maladie dentaire chez le chien. Pourtant caractérisée par une mauvaise haleine, elle est souvent non identifiée par le propriétaire. Sa prévention passe par des soins réguliers car elle peut entraîner la perte des dents voire des infections graves. La présence de bactéries dans la bouche est normale mais lorsqu'elles se développent trop rapidement, elles peuvent entraîner la formation de plaque dentaire. Si la plaque s'accumule et n'est pas éliminée, une gingivite (inflammation des gencives) peut apparaître. À ce stade, le traitement peut être complètement curatif. Cependant, en l'absence de traitement, la maladie évolue en périodontite caractérisée par une inflammation plus importante des gencives, des dépôts de tartre sur les dents et la disparition de l'os et des structures de soutien entourant la dent. L'atteinte peut être prise en charge mais est irréversible. La parodontite peut entraîner la perte des dents et la propagation d'infections graves au niveau du foie, du cœur ou des poumons.

Selon un aspect particulier de l'invention, les composés de formule (I) sont administrés en combinaison avec un autre composé antibiotique, notamment de la famille des bêtalactamines (pénicillines / céphalosporines), aminosides, macrolides, polypeptides, sulfamides, quinolones, nitro-imidazolés, dérivés des nitrofuranes, dérivés du noyau benzyl-pyrimidine, des tétracyclines ou des phénicolés, tels que la doxycycline ou le chloramphénicol, la pénicilline, l'ampicilline, amoxicilline, la cloxacilline, la dicloxacilline, l'oxacilline, la nafcilline, la céfalexine, la céfapirine, la céfazoline, le ceftiofur, la céfopérazone, la céfovécine, le cefquinome, la thimaphénicol, le florfénicol, la terramycine, l'érythromycine, la spiramycine, la tylosine, la josamycine, le tilmicosine, la tulathromycine, la gamithromycine, la tildipirosine, la clyndamycine, la lyncomycine, la pirlymicine, le tiamuline, la valnémuline, l'acide oxolinique, la fluméquine, l'enrofloxacine, la danofloxacine, l'ibafloxacine, la marbofloxacine, la difloxacine, l'obifloxacine, la pradofloxacine, la rifampicine, la rifaximine, le sulfaméthizol, la sulfathiazol, la sulfadimidine, la sulfaméthoxazole, la sulfadiazine, la sulfadiméthoxine, la sulfaméthoxypyridazine, le triméthoprime, la baquiloprime, le métronidazole, le dimétridazole, la ronidazole, la nitrofurantoïne, la furazolidone ou la furaltadone. Dans une utilisation particulièrement avantageuse des composés de l'invention, on observe une synergie lors de l'utilisation conjointe des composés de l'invention avec des antibiotiques. En effet, il a été observé que lorsque les composés de formule (I) étaient associés à un autre composé antibiotique, par exemple la doxycycline ou le chloramphénicol sur une souche Gram-négative de *Pseudonomas aeruginosa,* une synergie était observée. Cette propriété permet par exemple de traiter efficacement des patients avec un taux moindre d'antibiotique, ce qui peut diminuer l'apparition de résistance aux antibiotiques.

### Composition pharmaceutique

Selon un deuxième objet, l'invention concerne une composition pharmaceutique ou vétérinaire comprenant un composé de formule (I) et un excipient pharmaceutiquement acceptable : 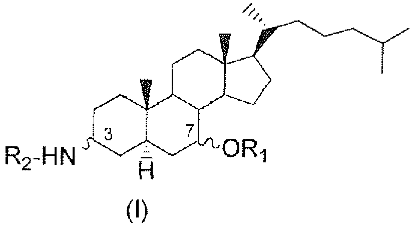 dans laquelle :
R₁, R₂, sont tels que définis ci-dessus,
ainsi que les stéréoisomères, mélanges de stéréoisomères et/ou les sels pharmaceutiquement acceptables des composés de formule (I).
Le composé 3β-(norspermine)-7α-hydroxy-5α-cholestane et les composés de formule suivante sont exclus :

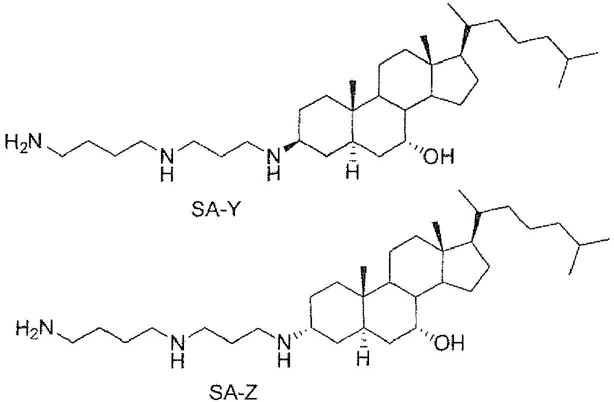

Selon un aspect de l'invention, il est fourni des compositions pharmaceutiques comprenant en outre un deuxième composé antibiotique, notamment de la famille des bêtalactamines (pénicillines / céphalosporines), aminosides, macrolides, polypeptides, sulfamides, quinolones, nitro-imidazolés, dérivés des nitrofuranes, dérivés du noyau benzyl-pyrimidine, des tétracyclines ou des phénicolés, ou un deuxième composé antiparasitaire, notamment antipaludéen.

Les compositions pharmaceutiques ou vétérinaires selon l'invention peuvent être présentées sous des formes solides ou liquides, destinées par exemple à l'administration par voie parentérale (intraveineuse, intramusculaire, sous cutanée), orale ou topique.

Elles seront donc présentées sous forme de solutés ou de suspensions injectables ou flacons mono-doses ou multi-doses, sous forme de comprimés nus ou enrobés, de dragées, de capsules, de gélules, de pilules, de cachets, de poudres, de granulés, de suppositoires ou de capsules rectales.

De façon avantageuse, le produit selon l'invention comprend également un ou plusieurs ingrédients additionnels bien connus de l'homme du métier tels que notamment, les agents liants, les agents de granulation, les lubrifiants, les colorants, les charges, les émulsifiants, les minéraux, les agents de pelliculage, les sels, les stabilisants, les tampons ou les vitamines. Les stabilisants comprennent les substances qui ont tendance à augmenter la durée de conservation de la composition tels que les conservateurs, les émulsifiants, les épaississants, les gaz d'emballage, les gélifiants, les humectants, les séquestrants, les synergistes ou les stabilisants.

Pour l'usage parentéral, l'eau, les solutés aqueux, le sérum physiologique, les solutés isotoniques sont les véhicules les plus commodément utilisés.

Pour l'usage percutané, notamment sur la peau, les muqueuses ou le poil, en particulier pour les solutions à verser de type « pour-on » ou « spot-on » en médécine vétérinaire, les excipients usuels sont des solvants aqueux, alcooliques, polaire ou non, qui favorisent le passage transcutané, tel que l'eau, l'alcool benzylique, les huiles végétales et minérales, les agents de remise en suspension, les antioxydants, les tensioactifs, notamment un mélange constitué d'alcool benzylique et/ou de labrasol et/ou de laurate de propylène glycol, à titre d'agent de pénétration peut être utilisé.

La posologie peut varier dans les limites importantes (0,05 mg à 1000 mg) en fonction de l'indication thérapeutique et de la voie d'administration, ainsi que de l'âge et du poids du sujet.

### Composés de formule (I)

Selon un troisième objet, l'invention concerne des composés de formule (I) : 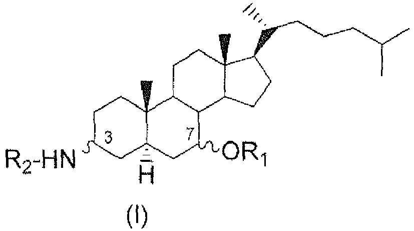 dans laquelle :
R₁, R₂, sont tels que définis ci-dessus,
ainsi que les stéréoisomères, mélanges de stéréoisomères et/ou les sels pharmaceutiquement acceptables des composés de formule (I).

Le composé 3β-(norspermine)-7α-hydroxy-5α-cholestane et les composés de formule suivante sont exclus :

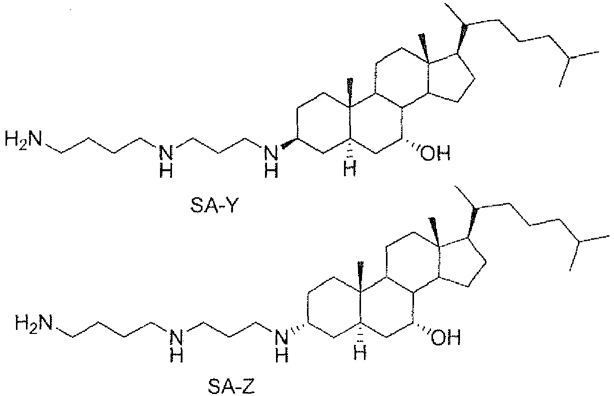

### Procédé de préparation des composés de formule (I)

Les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans les exemples qui suivent.

Selon un autre objet, la présente invention concerne donc également le procédé de préparation des composés de formule (I) précédemment décrits, comprenant une étape d'amination réductrice du composé de formule (II) en présence d'une amine R₂NH₂, ce par quoi on obtient le composé de formule (I) :

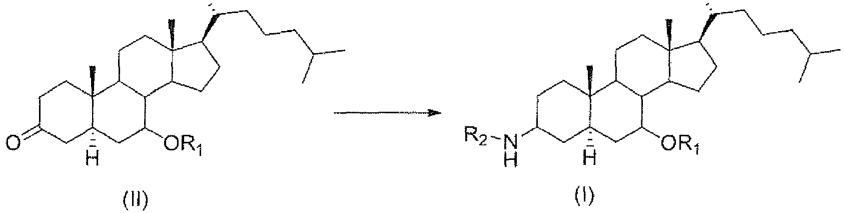

Eventuellement ledit procédé peut également comprendre l'étape consistant à isoler le produit obtenu.

Le composé ainsi préparé peut être récupéré à partir du mélange de la réaction par les moyens traditionnels. Par exemple, les composés peuvent être récupérés en distillant le solvant du mélange de la réaction ou si nécessaire après distillation du solvant du mélange de la solution, en versant le reste dans de l'eau suivi par une extraction avec un solvant organique immiscible dans l'eau, et en distillant le solvant de l'extrait. En outre, le produit peut, si on le souhaite, être encore purifié par diverses techniques, telles que la recristallisation, la reprécipitation ou les diverses techniques de chromatographie, notamment la chromatographie sur colonne ou la chromatographie en couche mince préparative.

Il sera apprécié que les composés utiles selon la présente invention peuvent contenir plusieurs centres asymétriques. Ces centres asymétriques peuvent être indépendamment en configuration R ou S. On doit comprendre que la présente invention comprend les stéréoisomères, incluant les énantiomères ou diastéréoisomères, et des mélanges de ceux-ci, incluant des mélanges racémiques, de composés de formule (I) ci-dessus. Ces stéréoisomères peuvent être séparés de leurs mélanges, par l'application ou l'adaptation de procédés connus, par exemple des techniques de chromatographie ou des techniques de recristallisation, ou ils peuvent préparés séparément à partir des stéréoisomères appropriés de leurs intermédiaires.

Les produits de bases ou les réactifs utilisés sont disponibles commercialement et/ou peuvent être préparés par l'application ou l'adaptation de procédés connus, par exemple des procédés tels que décrits dans les Exemples ou leurs équivalents chimiques évidents.

### Définitions

Selon la présente invention, les radicaux « alkyles » représentent des radicaux hydrocarbonés saturés, en chaîne droite ou ramifiée, de 1 à 8 atomes de carbone, notamment de 1 à 6 atomes de carbone, de préférence de 1 à 4 atomes de carbone. On peut notamment citer, lorsqu'ils sont linéaires, les radicaux méthyle, éthyle, propyle, butyle, pentyle, hexyle. On peut notamment citer, lorsqu'ils sont ramifiés, les radicaux isopropyle, tert-butyl, 2-méthylbutyle, 2-méthylpentyle, et 1-méthylpentyle.

Par groupe « aryle », on entend, au sens de la présente demande, un système aromatique hydrocarboné, mono ou bicyclique de 6 à 10 atomes de carbone. Parmi les radicaux aryles, on peut notamment citer le radical phényle ou naphtyle.

Par groupe « hétérocyclyle », on entend, au sens de la présente demande, un système hydrocarboné mono-, ou bicyclique, saturé, insaturé ou aromatique comprenant un ou plusieurs hétéroatomes tels que O, N ou S. Les groupes hétérocyclyles incluent notamment les groupes hétéroaryle ou hétérocycloalkyle.

Les groupes « hétéroaryles » désignent les systèmes aromatiques mono ou bicyclique, et ayant de 5 à 7 chaînons (atomes de cycle), notamment de 5 à 6 chaînons, comprenant un ou plusieurs hétéroatomes choisis parmi l'azote, l'oxygène ou le soufre,. Parmi les radicaux hétéroaryles, on peut citer l'imidazolyle, le pyrazinyle, le thiényle, l'oxazolyle, le furazanyle, le pyrrolyle.

Les radicaux « hétérocycloalkyles » désignent les systèmes mono ou bicycliques, saturés de 5 à 7 chaînons (atomes de cycle), notamment de 5 à 6 chaînons, comprenant un ou plusieurs hétéroatomes choisis parmi N, O ou S. Parmi les hétérocycloalkyles, on peut notamment citer la pyrazolidine, la pipéridine, la morpholine, la pipérazine.

L'expression « sels pharmaceutiquement acceptables » fait référence aux sels d'addition d'acides inorganiques et organiques, pharmaceutiquement acceptables, et les sels d'addition de bases pharmaceutiquement acceptables, des composés de la présente invention. Ces sels incluent des sels d'addition acides, c'est-à-dire des sels d'acide organique ou minéral d'un composé comportant une fonction basique telle qu'une amine, ou des sels d'addition basiques, c'est-à-dire des sels alcalins ou organiques d'un composé comportant une fonction acide telle qu'un acide carboxylique. Ces sels peuvent être préparés in situ pendant l'isolement final et/ou la purification des composés. En particulier, les sels d'addition acide peuvent être préparés en faisant réagir séparément le composé purifié avec un acide organique ou inorganique et en isolant le sel ainsi formé. Parmi les exemples de sels d'addition acide on trouve les sels bromhydrate, chlorhydrate, sulfate, bisulfate, phosphate, nitrate, acétate, oxalate, valerate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-b-hydroxynaphtoates, acide gentisique, iséthionates, di-p-toluoyltartrates, methanesulfonates, éthanesulfonates, benzenesulfonates, p-toluenesulfonates, cyclohexyl sulfamates et quinateslaurylsulfonate, et analogues. (Voir par exemple S.M. Berge et al. « Pharmaceutical Salts » J. Pharm. Sci, 66 :p.1-19 (1977)).

Les sels d'addition basiques peuvent également être préparés en faisant réagir séparément le composé purifié sous sa forme acide avec une base organique ou inorganique et en isolant le sel ainsi formé. Des exemples de sels d'addition basiques comprennent les sels de sodium, potassium, calcium, baryum, zinc, magnésium et aluminium. Les sels de sodium et de potassium sont préférés. Les sels d'addition basiques peuvent notamment être préparés à partir d'hydrures ou d'hydroxydes de métal alcalin ou alcalino-terreux qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc.

Tel qu'il est utilisé ici, le terme «pharmaceutiquement acceptable» se réfère à des composés, compositions et / ou formes de dosage qui sont, dans la portée d'un jugement médical valable, adapté pour une utilisation en contact avec les cellules des humains et des animaux inférieurs sans toxicité, irritation, réponse allergique indue et similaires, et sont proportionnés à un rapport avantage/risque raisonnable.

Tel qu'il est utilisé ici, le terme « stéréoisomère » se réfère aux isomères optiques liés aux deux atomes de carbone asymétriques situés en position 3 et en position 7 sur la formula (I), et inclut les énantiomères et diastéréoisomères de ces composés.

D'autres caractéristiques de l'invention apparaîtront dans les exemples qui suivent. Ces exemples sont donnés pour illustrer l'invention et ne sont pas destinés à être limitatifs de celle-ci.

### Exemples

### I. Synthèse des composés de formule (I)

Toutes les synthèses ont été réalisées avec des solvants purifiés selon les méthodes usuelles. Les réactifs commerciaux sont directement utilisés sans purification préalable.

Les structures chimiques synthétisées ont toutes été vérifiées par une analyse RMN du proton (¹H) et/ou du carbone (¹³C) dans du chloroforme deutéré CDCl₃ ou du méthanol deutéré CD₃OD sur un appareil de type Bruker AC 300. Les déplacements chimiques δ sont exprimés en ppm. Les fréquences d'enregistrements des noyaux ainsi que des références utilisées sont les suivantes :
RMN du ¹H : 300 MHz, Si(CH₃)₄
RMN du ¹³C : 75 MHz, Si(CH₃)₄

Les abréviations utilisées pour l'écriture du spectre ¹H sont les suivantes :
- s = singulet
- d = doublet
- t = triplet
- q = quadruplet
- m = massif

Les spectres de masse ont été réalisés au Spectropôle d'Aix-Marseille III. Ils sont effectués sur le produit sec et à l'aide d'un spectromètre triple Quadripôle API III Plus Sciex. L'échantillon est dissous dans 500 µL de CH₂Cl₂ puis dilué au 1/10⁴ dans une solution de MeOH à 3 mM d'acétate d'ammonium. La solution de l'extrait est introduite dans la source d'ionisation par infusion (pompe pousse-seringue Harvard Apparatus) à un débit de 5 µL/min.

Les composés de formule (I) ont été préparés selon le schéma réactionnel ci-dessous :

### Synthèse du 7-kétocholest-5-ene-3β-ol 1

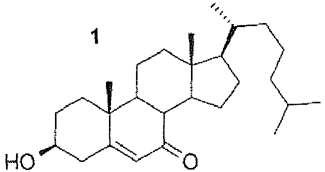

Dans un réacteur muni d'une agitation mécanique, on place 50 g de cholestérol (0,129 mol), 22 g d'hydroxyphtalamide (0,135 mol) et 1,5 L d'un mélange d'Acétate d'éthyle/Acétone (1/1). On porte à 50°C. On additionne 200 mg de peroxyde de benzoyle et on fait buller de l'air durant 72h en ajustant le niveau du solvant et en suivant la manipulation par chromatographie sur plaque. Après 72h, on évapore sous vide le solvant. Le résidu est dissout dans l'éther de pétrole et lavé avec du carbonate de sodium jusqu'à ce que la coloration orange disparaisse. Les phases organiques sont lavées avec une solution de NaCl saturée et séchées sur MgSO₄. Le solvant est chassé sous vide et le stérol est dissout dans la pyridine (200 mL). On refroidit à 0°C et on additionne 1 g de CuCl₂. La solution est agitée 24h (retour de 0°C à la température ambiante). La solution obtenue est jetée sur un mélange eau/glace. On extrait à l'acétate d'éthyle et on lave avec une solution de CuSO₄ saturée. Après séparation des phases, la phase organique est lavée avec une solution de HCl à 0,1N, et séchée sur MgSO₄. Après évaporation du solvant, le résidu est chromatographié sur gel de silice (Ether de pétrole/Acétate d'éthyle 1/1). La cétone attendue est obtenue sous la forme d'un solide blanc cassé avec un rendement de 70%.
RMN ¹H : δ = 5.45-5.75 (m, 1H), 3.475-3.75 (m, 1H), 2.125-2.75 (m, 4H), 1.75-2.075 (m, 6H), 0.8-1.7 (m, 37H), 0.45-0.75 (m, 4H) ; ¹³C : δ = 202.81, 165.59, 126.49, 70.89, 55.17, 50.34, 45.80, 43.49, 39.87, 38.67, 36.57, 36.11, 28.40, 26.72, 24.22, 23.22, 22.96, 21.61, 19.26, 17.71, 12.37.

### Synthèse du 7-kétocholest-5-ene-3β-acétate 2

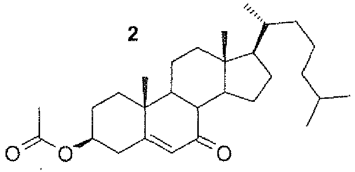

Dans un ballon bicol munis d'un réfrigérant, on dissout 3 mmol de 7-kétocholest-5-ene-3βol 1 dans de la pyridine (25 mL) et on additionne 9 mmol d'anhydride acétique. On laisse sous agitation magnétique pendant 24h dans un bain de glace. On évapore sous vide poussé la pyridine, puis on remet en suspension le solide obtenue dans du CH₂Cl₂ (15 mL). Une extraction au sulfate de cuivre est alors réalisée, et la phase organique est séchée sur MgSO₄, filtrée puis séchée sous vide poussé. On purifie par chromatographie le produit sur gel de silice (éluant : éther de pétrole/ acétate d'éthyle 9/1) (Rdt 94%).
RMN ¹H : δ = 5.65-5.7 (d, 1H), 4.6-4.75 (m,1H), 0.6-2.6 (m, 44H); ¹³C : δ = 201.90, 170.24, 163.81, 126.68, 72.19, 54.75, 49.93, 49.78, 45.38, 43.08, 39.44, 38.64, 38.28, 37.72, 36.15, 35.98, 35.69, 31.90, 28.50, 27.96, 27.32, 26.28, 23.80, 22.77, 22.52, 21.24, 21.14, 18.84, 17.22, 11.93.

### Synthèse du 7-kétocholestane-3β-acétate 3

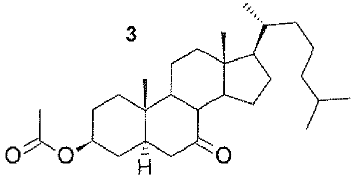

Dans un réacteur en acier, on introduit 3.04 g (6.87 mmol) de 7-kétocholestane-3βol 2 et 1.1 g (environ 15% mol) de Pd/C (10%) dans 50 mL de CH₂Cl₂. On place le réacteur sous 50 bars d'hydrogène et sous agitation forte durant 12h. Après filtration sur Célite et évaporation sous vide du solvant le produit est obtenu quasi pur et avec un rendement quantitatif. Il sera utilisé tel quel dans l'étape suivante.
RMN ¹H : δ = 4.62-4.70 (m,1H), 0.64-2.39 (m, 47H); ¹³C : δ = 211.44, 170.38, 72.71, 54.95, 49.90, 48.82, 46.43, 45.34, 42.45, 39.42, 38.65, 36.09, 35.89, 35.60, 33.79, 28.35, 27.93, 26.34, 24.93, 23.72, 22.74, 22.51, 22.15, 21.27, 18.73, 12.01, 11.65.

### Synthèse du mélange de 7β-hydroxycholestane-3β-ol 4a et 7α-hydroxycholestane-3β-ol 4b

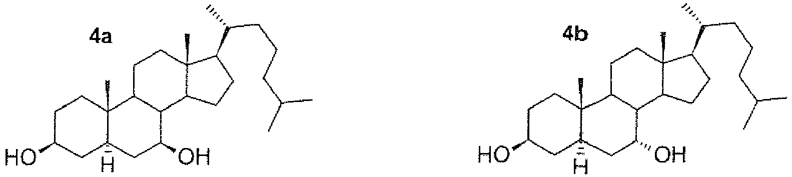

Dans un ballon bicol on place 530 mg d'hydrure de lithium aluminium (13.8 mmol) dans 100 mL de THF anhydre. On additionne lentement à 0°C une solution de 7-kétocholestane-3β-acétate 3 (1.5g, 3.3 mmol) dissout dans 15 mL de THF. Après 12 h d'agitation à température ambiante, on hydrolyse avec une solution de KOH (30%) (1.2 mL). On poursuit l'agitation 1h puis on filtre sur Célite, on rince au MeOH puis on évapore les solvants sous vide. On purifie par chromatographie le produit sous la forme d'un mélange d'isomères (α/β 50/50) sur gel de silice (éluant : éther de pétrole/ acétate d'éthyle 8/2) (Rdt 95%).
RMN ¹H : δ = 0.6-3.82 (m, 48H); ¹³C : δ = 75.11, 71.08, 70.99, 67.96, 62.57, 56.45, 56.08, 55.68, 55.19, 51.85, 50.51, 45.83, 43.95, 43.35, 42.60, 42.00, 39.46, 37.62, 37.11, 36.54, 36.24, 35.73, 35.51, 34.88, 31.30, 29.82, 28.05, 27.95, 26.86, 23.79, 23.71, 22.76, 22.51, 21.56, 20.96, 18.60, 12.40, 12.11, 11.79, 11.20.

### Synthèse du 3-kéto-7β-hydroxycholestane 5a et 3-kéto-7α-hydroxycholestane 5b

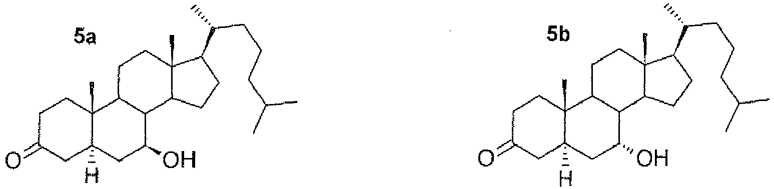

Dans un ballon monocol surmonté d'un Dean-Stark on place 1.28 g de mélange de 7β-hydroxycholestane-3β-ol 4a et 7α-hydroxycholestane-3β-ol 4b dans 150 mL de toluène et 3.6 g de carbonate d'argent sur Célite. On porte à reflux du toluène durant 24h. Après refroidissement le mélangest filté sur Célite. On purifie par chromatographie sur gel de silice (éluant : éther de pétrole/ acétate d'éthyle 7/3) et l'on obtient ainsi les deux isomères purs 5b (fraction 1)et 5a (fraction 2) avec des rendements non optimisés de 31 et 25 % respectivement.
5a RMN ¹H : δ = 3.05-3.55 (m, 1H), 2.2-2.6 (m, 3H), 0.4-1.92 (m, 42H) ; ¹³C : δ = 211.55, 74.66, 55.61, 55.24, 51.83, 44.18, 43.94, 43.66, 39.90, 39.54, 38.12, 35.72, 35.16, 28.75, 28.06, 23.89, 22.87, 22.62, 21.80, 18.84, 12.22, 11.63.
5b RMN ¹H : δ = 3.3-3.85 (m, 1H), 0.60-2.65 (m, 45H) ; ¹³C: δ = 211.36, 71.08, 57.18, 56.53, 54.33, 47.13, 39.86, 38.32, 37.07, 36.49, 36.08, 31.10, 30.44, 28.43, 28.39, 24.38, 24.20, 23.20, 22.93, 21.92, 19.03, 13.53, 12.40.

**Synthèse des dérivés aminostéroïdiens SA1-SA12** (les dérivés SA-3 et SA-10 ne font pas partie de la présente invention).

Les dérivés aminostéroïdiens ont tous été produits selon le même mode opératoire. Considérons l'exemple de la molécule **SA-1.**

Dans un ballon bicol mis sous argon, 3.3 équivalents de spermine (171 mg, 0,82 mmol) sont dissous dans 5 mL de MeOH, puis on additionne 300 µL de Ti(O*i*-Pr)₄ (1 mmol). Après 5 minutes d'agitation, on ajoute au mélange 102 mg de 3-kéto-7α-hydroxycholestane 5b (0,25 mmol). Après 24 heures sous agitation, on place le ballon à - 78°C, puis 40 mg de NaBH₄ (1 mmol) sont ajoutés sous agitation. Après 2 heures et retour à température ambiante, 1 mL d'eau est ajouté pour terminer la réaction. Après 1h supplémentaire d'agitation, le mélange est filtré sur Célite. Le filtrat est évaporé sous vide le produit est purifié par chromatographie sur gel de silice (éluant : CH₂Cl₂/MeOH/NH₄OH (7/3/1)).

### 3β-spermino-7α-hydroxy-5α-cholestane SA-1

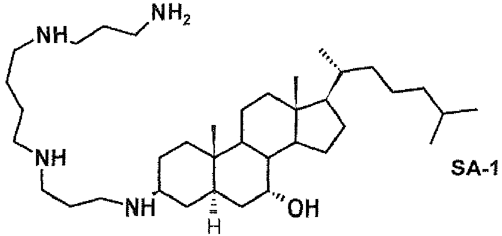

Rdt: 54%. RMN ¹H : δ = 3.16 (m, 1H), 2.44-2.65 (m,13H), 1.96 (m, 5H), 0.89-1.64 (m, 57H); ¹³C : δ = 71.65, 57.46, 56.03, 51.04, 47.98, 47.64, 46.61, 45.34, 42.76, 40.80, 40.60, 40.02, 39.82, 39.74, 38.62, 37.16, 34.58, 33.12, 29.45, 28.30, 28.12, 25.11, 24.30, 24.28, 22.68, 20.91, 18.73, 13.56, 11.92.

### 3β-spermino-7β-hydroxy-5α-cholestane SA-2

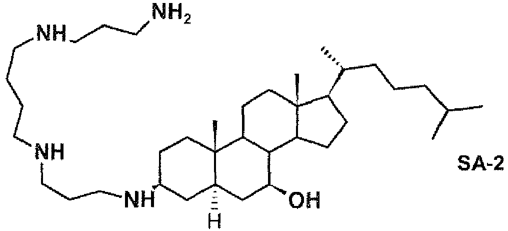

Rdt: 49%. RMN ¹H : δ = 3.05 (m, 1H), 2.44-2.65 (m,13H), 1.95 (m, 5H), 0.89-1.63 (m, 57H); ¹³C : δ = 73.95, 57.46, 56.03, 51.04, 47.98, 47.64, 46.84, 45.84, 42.66, 41.80, 40.75, 40.39, 39.82, 39.65, 37.45, 37.26, 36.28, 26.26, 34.58, 33.97, 29.45, 28.30, 28.08, 25.35, 25.12, , 24.30, 24.28, 21.98, 20.90, 18.73, 13.54, 11.89.

### 3β-Norspermidino-7β-hydroxy-5α-cholestane SA-3

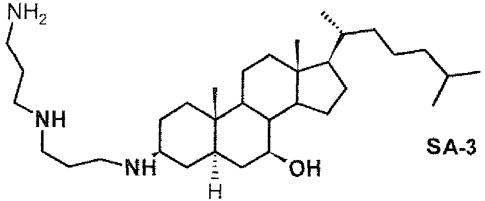

Rdt: 46%. RMN ¹H : δ = 3.14 (m, 1H), 2.55-2.65 (m,8H), 2.01 (m, 4H), 1.01-1.83 (m, 50H); ¹³C : δ = 73.46, 57.64, 56.03, 52.39, 47.47, 46.54, 45.98, 42.79, 41.20, 40.78, 40.54, 40.22, 40.10, 37.12, 37.02, 36.28, 36.26, 34.58, 33.91, 29.41, 29.12, 28.23, 25.62, 23.41, 23.12, 22.68, 20.84, 18.74, 13.68, 11.97.

### 3β-(1,3-diaminopropane)-7β-hydroxy-5α-cholestane SA-4

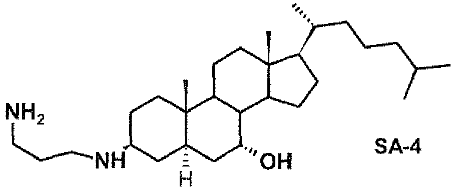

Rdt: 54%. RMN ¹H : δ = 3.21 (m, 1H), 2.53-2.60 (m, 4H), 0.89-1.81 (m, 51H); ¹³C : δ = 71.22, 57.33, 55.89, 52.02, 46.01, 41.78, 40.96, 40.35, 39.92, 39.25, 39.02, 37.98, 37.11, 36.58, 36.45, 34.75, 34.25, 29.68, 28.64, 28.02, 23.56, 23.12, 22.68, 20.87, 18.98, 14.02,11.92.

### 3β-(1,4-diaminobutane)-7β-hydroxy-5α-cholestane SA-5

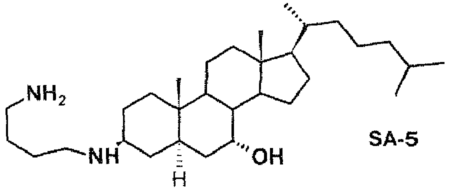

Rdt: 48%. RMN ¹H : δ = 3.17 (m, 1H), 2.55-2.63 (m,4H), 0.99-1.83 (m, 53H); ¹³C : δ = 71.23, 56.03, 55.66, 51.02, 48.29, 42.78, 41.19, 40.98, 40.02, 39.74, 39.65, 38.54, 37.14, 36.54, 36.45, 35.02, 34.89, 29.54, 29.45, 28.30, 28.08, 25.33, 24.85, 24.12, 22.67, 20.91, 18.76, 13.56, 11.90.

### 3β-(Tris(2aminoethyl)amine)-7β-hydroxy-5α-cholestane SA-6

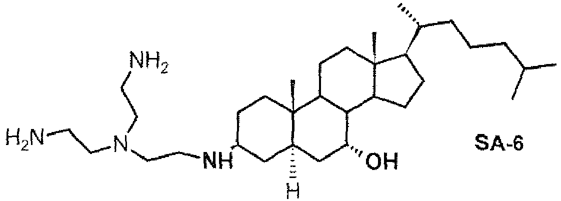

Rdt: 29%. RMN ¹H : δ = 3.12 (m, 1H), 2.42-2.75 (m,10H), 1.89 (m, 6H), 1.01-1.87 (m, 47H); ¹³C : δ = 71.35, 57.70, 56.32, 55.24, 54.44, 51.04, 43.59, 42.98, 40.80, 40.12, 40.02, 40.00, 38.88, 38.32, 36.27, 35.97, 35.85, 35.02, 34.87, 29.18, 28.30, 28.08, 24.22, 23.85, 22.67, 20.89, 18.79, 13.54, 11.94.

### 3β-(1,5-diaminopentane)-7α-hydroxy-5α-cholestane SA-7

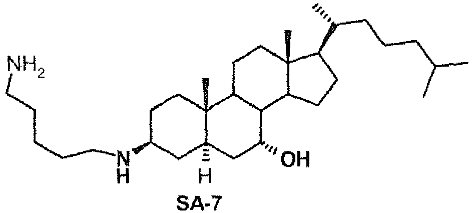

Rdt: 32%. RMN ¹H : δ = 3.14 (m, 1H), 2.54-2.60 (m,5H), 0.95-1.89 (m, 54H); ¹³C : δ = 71.90, 56.87, 52.43, 51.92, 48.71, 47.38, 43.37, 42.57, 41.02, 40.51, 39.71, 36.81, 36.51, 36.46, 36.38, 34.38, 34.12, 32.10, 29.84, 29.34, 28.68, 24.89, 22.44, 18.56, 12.34.

### 3β-(1,4-bis(3-aminopropyl)piperazine)-7β-hydroxy-5α-cholestane SA-8

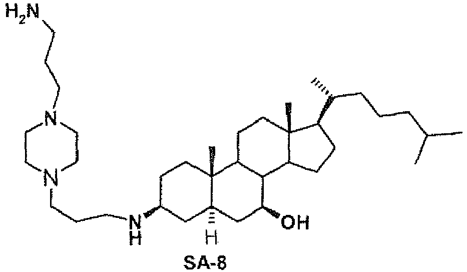

Rdt: 48%. RMN ¹H : δ = 3.21 (m, 1H), 2.52-2.66 (m,6H), 0.97-1.95 (m, 63H); ¹³C : δ = 73.12, 56.42, 52.4, 51.91, 48.70, 47.32, 43.35, 42.55, 41.0, 40.57, 39.77, 36.89, 36.59, 36.40, 36.34, 34.38, 34.14, 32.12, 29.85, 29.37, 28.62, 24.88, 22.41, 22.41, 18.13, 12.33.

### 3β-(1,4-bis-(3-aminopropoxy)butane)-7β-hydroxy-5α-cholestane SA-9

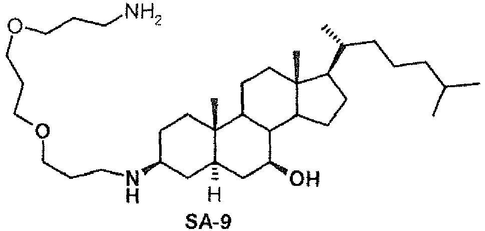

Rdt: 42%. RMN ¹H : δ = 3.13 (m, 1H), 2.51-2.60 (m,4H), 0.97-1.98 (m, 63H); ¹³C : δ = 73.80, 68.33, 68.12, 66.44, 66.06, 57.23, 51.95, 51.72, 48.84, 43.35, 43.02, 41.92, 41.63, 39.32, 38.25, 36.45, 35.77, 35.62, 35.12, 34.80, 33.42, 33.12, 29.87, 28.75, 26.33, 26.01, 22.12, 21.84, 18.63, 11.92.

### 3β-(norspermine)-7α-hydroxy-5α-cholestane SA-10

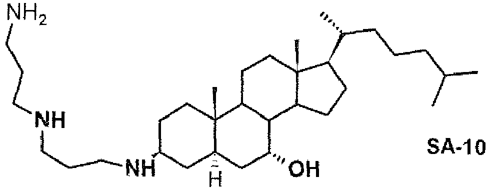

Rdt: 48%. RMN ¹H : δ = 3.13 (m, 1H), 2.55-2.66 (m,8H), 1.01-2.02 (m, 54H); ¹³C : δ = 71.9, 57.64, 56.03, 52.39, 47.50 46.54, 45.98, 42.79, 41.20, 40.78, 40.54, 40.22, 40.10, 37.12, 37.02, 36.27, 36.26, 34.58, 33.81, 29.41, 29.12, 28.23, 25.62, 23.41, 23.12, 22.67, 20.84, 18.04, 13.50, 11.87.

### 3β-(1-(3-aminopropyl)imidazole)-7α-hydroxy-5α-cholestane SA-11

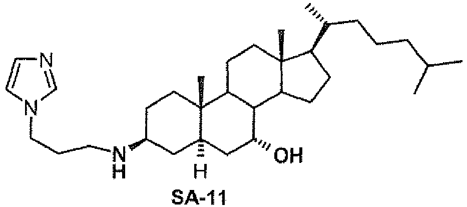

Rdt: 22%. RMN ¹H : δ = 3.18 (m, 1H), 2.56-2.66 (m,4H), 1.09-2.01 (m, 42H); ¹³C : δ = 139.81, 126.32, 123.27, 71.68, 57.28, 52.39, 47.57 46.78, 45.98, 42.79, 41.14, 40.88, 40.14, 40.02, 40.00, 37.12, 37.02, 36.26, 36.26, 34.53, 33.82, 29.41, 29.12, 28.23, 25.62, 23.41, 23.12, 22.66, 20.84, 18.14, 13.50, 11.87.

### 3β-(1-(3-aminopropyl)morpholine)-7α-hydroxy-5α-cholestane SA-12

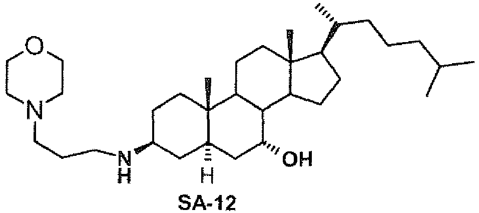

Rdt: 34%. RMN ¹H : δ = 3.14 (m, 1H), 2.56-2.66 (m,6H), 0.94-2.02 (m, 55H); ¹³C : δ = 72.01, 71.09, 70.95, 55.44, 55.12, 55.03, 52.39, 47.42 47.24, 45.88, 42.19, 41.00, 40.87, 40.54, 40.22, 40.11, 37.12, 37.02, 36.27, 36.26, 35.58, 34.81, 29.41, 29.72, 28.21, 25.62, 23.41, 23.03, 22.87, 21.82, 19.04, 13.26, 11.19.

### II. Evaluation des activités biologiques

### A. Activités antibactériennes intrinsèques des composés de formule (I)

### 1) Préparation de la préculture

Deux tubes ont été préparés :
Un témoin négatif (2 mL de milieu de culture stérile)
Un témoin positif (1940 µL de milieu de culture + 40µL de DMSO + 20 µL de la suspension bactérienne) à partir d'une souche biologique décongelée (La conservation des souches biologiques est réalisée à - 80°C dans du glycérol). Les souches utilisées sont S. *aureus* ATCC 25923, *E. coli* ATCC 25922, *P. aeruginosa* ATCC 27853, *C. albicans* CIP 1180-79 et *E. faecalis* CIP 103015.

Les tubes ont été incubés dans un infors à 37° C pendant 24 heures à 100 tours/minute.

Les germes ont été manipulés sous une hotte dans le laboratoire de type L2 et avant toute manipulation un cycle d'UV a été programmé et seul du matériel stérile a été utilisé. Un test de toxicité des solvants (méthanol, éthanol, DMSO) a été réalisé et ces derniers se sont montrés non toxiques à des concentrations inférieures ou égales à 2%. Les molécules chimiques à tester ont été préparées dans un mélange de DMSO/méthanol (50/50) à une concentration de 5 mg/mL.

### 2) Préparation de la microplaque pour la détermination de la concentration minimale d'inhibition (CMI)

Après 24 h d'incubation, une mesure de la densité optique a été effectuée à l'aide d'un spectrophotomètre à 600 nm en prélevant 100µL de la suspension bactérienne diluée dans 900 µL du milieu de culture stérile. Ce test a nécessité l'utilisation d'une plaque de 96 puits et le volume nécessaire de la suspension microbienne à ensemencer a été calculé pour une DO correspondante à une valeur égale à 0.01 dans chaque puit. Dans cette plaque, la première ligne correspondait au témoin négatif (195 µL de milieu de culture stérile dans chaque puit), la deuxième ligne au témoin positif (milieu de culture ensemencé et additionné de 2 % de DMSO), la troisième ligne a été chargée deux fois en suspension bactérienne, 8 µL de produit à tester ont été placés dans chaque puit. Par la suite, une dilution en cascade au demi a été réalisée à partir de cette ligne.

La première colonne a servi comme témoin d'inhibition. Un filtre stérile a ensuite été placé sur la microplaque permettant le passage des gaz mais pas des contaminants. La microplaque a été incubée à 37° C dans une atmosphère humide durant 24h.

**NB:** Le milieu utilisé est le milieu Mueller-Hinton (MH) pour les bactéries. Tous les tests ont été réalisés en duplicate.

### 3) Lecture des résultats

Après incubation, le filtre a été remplacé par un film transparent, ensuite une lecture de DO a été réalisée dans un spectrophotomètre à plaques IEMS à 620 nm. Un calcul de la concentration minimale d'inhibition (**CMI**) a été réalisé.

**Tableau I: Activités antibactériennes intrinsèques des composés de formule (I)**

| Compounds | CMI (µg/mL) | | | | | |
|---|---|---|---|---|---|---|
| | *S. aureus* ATCC 25923 | *S. aureus* Methicilline resistant | *S. faecalis* | C. albicans | *E. coli* ATCC 25922 | *P. aeruginosa* ATCC 27853 |
| Squalamine | 4 | 4 | 4 | 2 | 12 | 20 |
| SA-1 | 4 | 2 | 2 | 2 | 16 | 32 |
| SA-2 | 4 | 2 | 2 | 2 | 32 | 32 |
| SA-3 | 5 | 2,5 | 2,5 | 2,5 | 20 | 40 |
| SA-4 | 4 | 2 | 2 | 2 | 32 | 32 |
| SA-5 | 4 | 4 | 4 | 4 | 32 | 32 |
| SA-6 | 5 | 5 | 2,5 | 2,5 | 20 | 40 |

### B. Comparaison de la cytotoxicité et des activités antibactériennes des composés de formule (I) avec celles des composés (IIa), (IIb), (IIe) et (IId) divulgués dans la demande WO 2011/067501

Le test WST1 a été utilisé pour mesurer l'activité cytotoxique des produits. C'est un test colorimétrique qui permet de mesurer la viabilité et le taux de prolifération cellulaire. Il est basé sur le clivage de sels de tétrazolium incolores WST-1 (4-[3-(4-iodophényl)-2-(4-nitrophényl)-2H-5-tétrazolio]-1,3-benzène disulfonate) par les déshydrogénases mitochondriales en dérivé formazan de couleur jaune, quantifiables par spectrophotométrie à 420-480 nm.

Le test WST1 a été effectué sur des cellules d'ovaires de hamster chinois. Les cellules CHO-K1 (ATCC, USA) sont maintenues en culture dans du milieu Mac Coy's 5A additionné de 10% de sérum de veau fœtal, de 2 mM de L-glutamine et d'un mélange de pénicilline streptomycine (100 U/ml : 10 µg/ml). On incube à 37°C sous atmosphère enrichie en CO₂ (5%) et repiquées tous les deux jours.

Les cellules sont transférées dans des plaques de 96 puits (25000 cellules/mL) dans du milieu de Mc Coy's 5A complet, et maintenues pendant 24 h à 37°C sous atmosphère humide enrichie en CO₂ (5%). Des concentrations croissantes de produits à tester sont ajoutées dans les puits en doubles essais et 8 témoins de croissance contenant les cellules dans le milieu seul sont inclus dans chaque série de tests. Après 24 heures à 37°C (5% de CO₂), le milieu de culture est éliminé, les cellules sont rincées dans du tampon phosphate (PBS) et 50 µL de PBS contenant 10% de réactif WST1, sont additionnés dans chaque puits. Après 20 minutes d'incubation à 37°C, la lecture des résultats s'effectue par spectrophotométrie à 450 nm.

Les résultats sont exprimés sous forme de relations dose-réponse, modélisés par une analyse de régression non-linéaire à l'aide du logiciel TableCurve. La Concentration Inhibitrice 50% (CI₅₀) représente la concentration en produit capable de réduire de 50% la viabilité cellulaire.

Les composés (IIa), (IIb), (IIc) et (IId) suivants ont été préparés en suivant le protocole de synthèse divulgué dans la demande WO 2011/067501 :

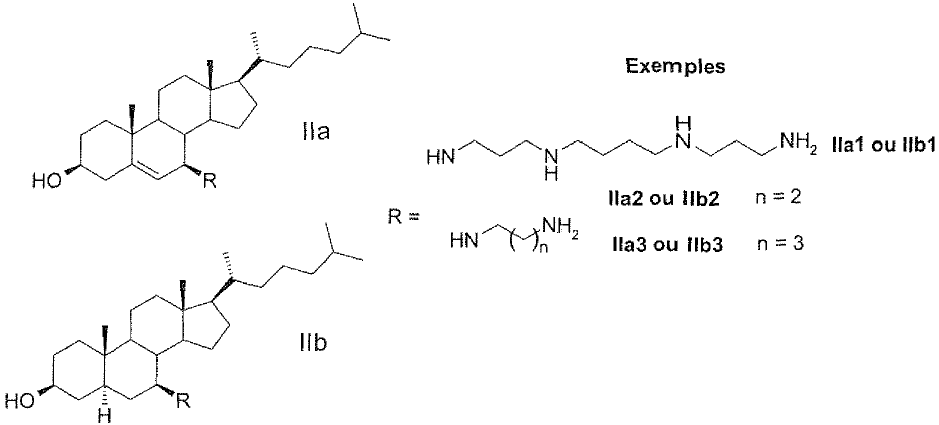

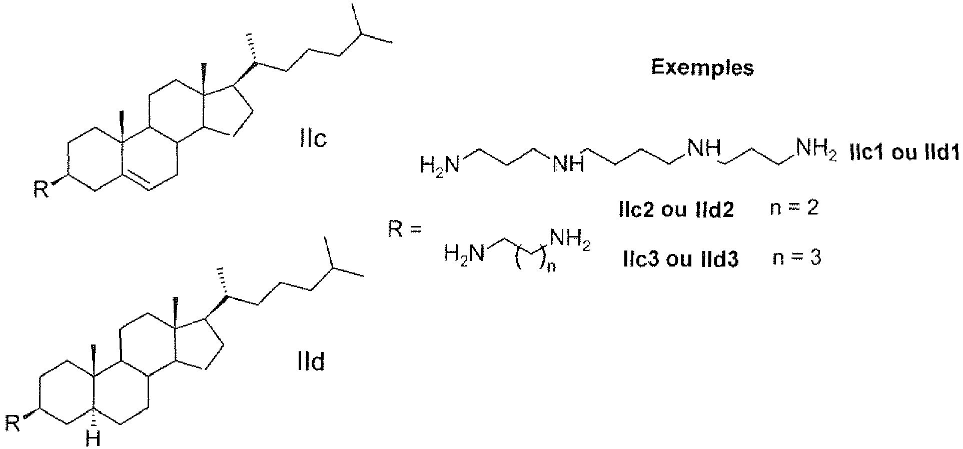

Les concentrations minimales inhibitrices (CMI) et la cytotoxycité (IC₅₀) ont été évaluées selon les protocoles expérimentaux ci-dessus.

**Tableau II : Comparaison des activités antibactériennes intrinsèques des composés de formule (I) selon l'invention avec celles des composés de l'état de la technique**

| **Composé** | CMI (µg/mL) | | IC₅₀ CHO (µM) |
|---|---|---|---|
| | *S. aureus (Methicilline resistant)* | *E. coli* | |
| **IIa1 ou IIb1** | 5 | 10 | <5 |
| **IIa2 ou IIb2** | 3.125 | 3.125 | <5 |
| **IIa3 ou IIb3** | 3.125 | 3.125 | <5 |
| **IIc1 ou IId1** | 3.125 | >50 | <5 |
| **IIc2 ou IId2** | 12.5 | >50 | <5 |
| **IIc3 ou IId3** | 12.5 | >50 | <5 |
| **SA1** | 2 | 16 | 15 |
| **SA2** | 2 | 32 | 50 |
| **SA3** | 2.5 | 20 | 32 |
| **SA4** | 2 | 32 | 9 |
| **SA5** | 4 | 32 | 15 |
| **SA6** | 5 | 20 | 46 |

On constate que les composés de formule (I) sont moins cytotoxiques que les composés IIa, IIb, IIc, IId testés et présentent une activité antibactérienne supérieure à celle des composé IIc et IId sur les bactéries gram-négatives *E. coli.*

### C. Potentialisation de l'activité des antibiotiques classiques en présence des dérivés aminostéroïdiens de formule (I)

### Exemple de préparation de la microplaque pour la détermination de la concentration minimale d'inhibition (CMI) de doxycycline en présence d'un dérivé aminostéroïdien

Cette méthode nécessite l'utilisation d'une plaque de 96 puits, 100 µL d'un milieu de culture liquide est déposé dans chaque puit puis ensemencé avec la suspension microbienne précédemment préparée. Le volume nécessaire à ensemencer est calculé pour une DO de 0.01 qui correspond à environ 5.10⁶ bactéries dans chaque puit. Dans cette plaque, la première ligne correspond à un témoin négatif (200 µL de milieu de culture stérile dans chaque puit), la deuxième ligne à un témoin positif (100 µL de milieu de culture stérile + 100 µL de la suspension bactérienne), la troisième ligne contient 192 µL de milieu de culture, 8 µL de produit aminostéroïdien à tester sont placés dans chaque puit. Par la suite, une dilution en cascade est réalisée à partir de cette ligne. On additionne ensuite dans chaque puit des lignes 3 à 8 8 µL d'une solution de doxycycline (1 mg dissout dans 20 mL) pour obtenir un concentration finale en antibiotique de 2µg/mL. On additionne ensuite 92 µL de suspension bactérienne dans les lignes 3 à 8. La lecture des résultats (la détermination de la CMI (2µg/mL de doxyccyline) en présence de X µg/mL de dérivé aminostéroïdien) se fait après 24h d'incubation à 37° dans une atmosphère humide. Après 24 h d'incubation à 37°C, 40 µL d'iodure de nitro tetrazolium sont rajoutés dans chaque puit permettant de révéler la présence de bactéries vivantes en colorant le milieu en rose.

Le but a été dans ces premiers essais de mettre en évidence ou non une synergie des dérivés aminostéroïdiens en présence de faible concentration de deux antibiotiques classiques : la doxycycline et le chloramphénicol

Il faut tout d'abord mentionner que sur la souche Gram-négative de *P. aeruginosa* (PAO1), la doxycycline possède une CMI de 40 µg/mL et le chloramphénicol une CMI de 1024 µg/mL

L'utilisation de faibles quantités de dérivés aminostéroïdiens permet de restaurer (de réduire) la concentration nécessaire en antibiotique pour tuer la souche considérée. Les résultats sont consignés dans le tableau ci-après :

**Tableau III : Potentialisation de l'activité des antibibiotiques classiques en présence des dérivés aminostéroïdiens de formule (I)**

| Composés | Concentration en composé nécessaire pour restaurer l'activité de 1 antibiotique considéré (µg/mL) | |
|---|---|---|
| | Concentration doxycycline utilisée (2 µg/mL) | Concentration chloramphénicol utilisée (4 µg/mL) |
| Squalamine | 1,75 | 1,75 |
| SA-1 | 4 | 16 |
| SA-2 | 4 | 16 |
| SA-3 | 5 | 10 |
| SA-6 | 2,5 | 20 |

On constate une très bonne synergie de certains composés avec la doxycycline restaurant ainsi l'activité de cet antibiotique à de faibles concentrations d'utilisation (2 µg/mL). Dans le cas du chloramphénicol les résultats sont également encourageants puisque la CMI passe de 1024 à 4 µg/mL pour d'assez faibles doses de composés à ajouter.

### Références

(1) Alhanout K, Brunel JM, Raoult D, Rolain JM. In vitro antibacterial activity of aminosterols against multidrug-resistant bacteria from patients with cystic fibrosis. J Antimicrob Chemother 2009 Oct; 64(4):810-4.
(2) Loncle et al. Tetrahedron 2007, 63, 12968-12974.
(3) Salmi et al. European Journal of Medicinal Chemistry 2008, 43, 540-547.
(4) Salmi et al. Letters in Drug Design & Discovery 2008, 5, 169-172.
(5) Salmi et al. Journal of Enzyme Inhibition and Medicinal Chemistry 2008, 23, 860-865.
(6) Loncle et al. Letters in Drug Design & Discovery 2008, 5, 388-393.
(7) Salmi et al. Tetrahedron 2008, 64, 4453-4459.
(8) Williams JI et al. Squalamine treatment of human tumors in nu/nu mice enhances platinum-based chemotherapies. Clin Cancer Res. 2001 Mar;7(3):724-733.
(9) Daniel Amsterdam. Susceptibility testing of antimicrobials in liquid media. In Antibiotics in Laboratory Medicine. 5th Edition. Editor Victor Lorian. Lippincott Williams and Wilkins 2005. Philadelphia, USA.

## Revendications

1. Composé de formule (I) : 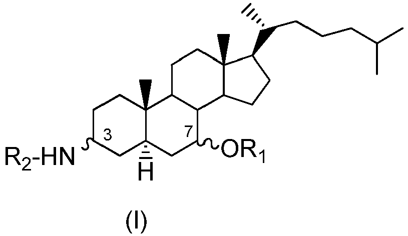 dans laquelle :
R₁ est choisi parmi H, SO₃H, un groupe alkyle en C₁-C₈, un groupe aryle en C₆-C₁₀, un groupe C(=O)R₁₁,
R₁₁ est choisi parmi un groupe alkyle en C₁-C₈, un groupe aryle en C₆-C₁₀,
le groupe -NHR₂ est choisi parmi : 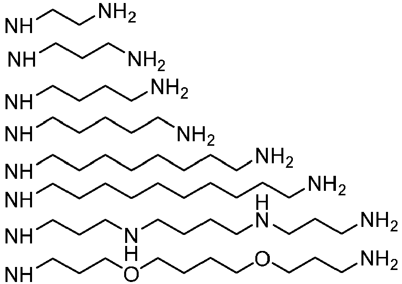 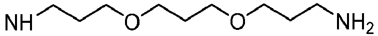 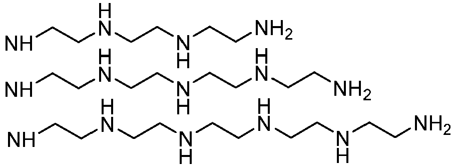 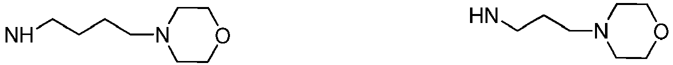 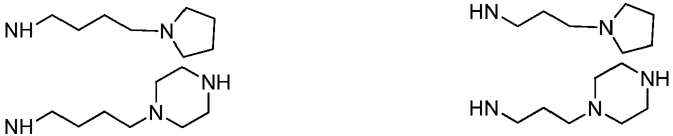 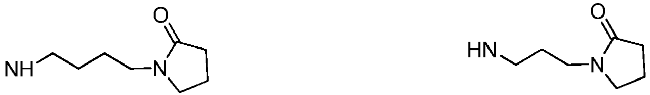 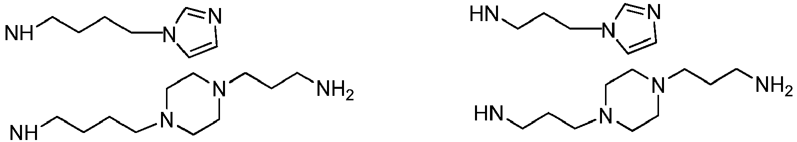 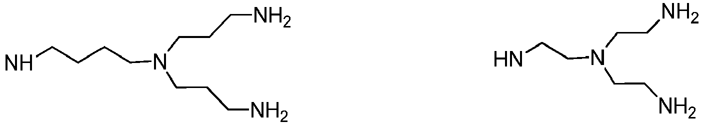
ainsi que les stéréoisomères, mélanges de stéréoisomères, et/ou sels pharmaceutiquement acceptables de celui-ci, pour leur utilisation dans le traitement des infections bactériennes ou fongiques chez l'homme ou l'animal.

2. Composé de formule (I) pour son utilisation selon la revendication 1, dans laquelle R₁ est H.

3. Composé de formule (I) pour son utilisation selon la revendication 1, choisi parmi :
- 3β-spermino-7α,-hydroxy-5α-cholestane (SA-1)
- 3β-spermino-7β-hydroxy-5α-cholestane (SA-2)
- 3β-(1,3-diaminopropane)-7β-hydroxy-5α-cholestane (SA-4)
- 3β-(1,4-diaminobutane)-7β-hydroxy-5α-cholestane (SA-5)
- 3β-(Tris(2aminoethyl)amine)-7β-hydroxy-5α-cholestane (SA-6)
- 3β-(1,5-diaminopentane)-7α-hydroxy-5α-cholestane (SA-7)
- 3β-(1,4-bis(3-aminopropyl)piperazine)-7β-hydroxy-5α-cholestane (SA-8)
- 3β-(1,4-bis-(3-aminopropoxy)butane)-7β-hydroxy-5α-cholestane (SA-9)
- 3β-(1-(3-aminopropyl)imidazole)-7α-hydroxy-5α-cholestane (SA-11)
- 3β-(1-(3-aminopropyl)morpholine)-7α-hydroxy-5α-cholestane (SA-12)

4. Composé de formule (I) pour son utilisation selon l'une quelconque des revendications précédentes, dans laquelle l'infection est une infection bactérienne, notamment à bactéries gram + ou gram -.

5. Composé de formule (I) pour son utilisation selon l'une quelconque des revendications précédentes, chez l'animal, notamment chez le chien, le chat, ou les ruminants.

6. Composé de formule (I) pour son utilisation selon l'une quelconque des revendications précédentes, pour le traitement de mammites, métrites, infections dentaires, pyodermites ou otites chez l'animal.

7. Composé de formule (I) pour son utilisation selon l'une quelconque des revendications précédentes, dans laquelle le composé de formule (I) est administré en combinaison avec un autre composé antibiotique.

8. Composition pharmaceutique ou vétérinaire comprenant un composé de formule (I) ou un stéréoisomère, un mélange de stéréoisomères et/ou un sel pharmaceutiquement acceptable d'un composé de formule (I) selon la revendication 1 et un excipient pharmaceutiquement acceptable.

9. Composition pharmaceutique ou vétérinaire selon la revendication 8, comprenant en outre un deuxième composé antibiotique.

10. Composé de formule (I) telle que définie à la revendication 1.

11. Procédé de préparation d'un composé de formule (I) selon la revendication 1, comprenant une étape d'amination réductrice d'un composé de formule (II) en présence d'une amine R₂NH₂, ce par quoi on obtient le composé de formule (I) : 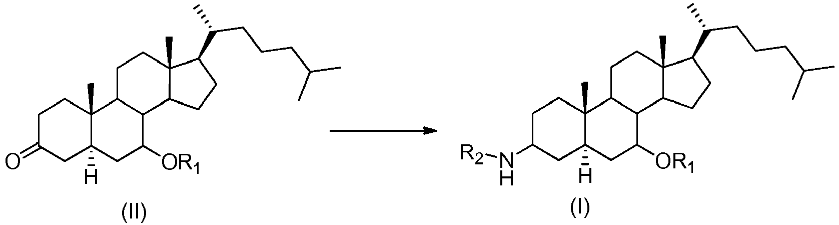 R₁, R₂ étant tels que définis dans la revendication 1.

## Patentansprüche

1. Verbindung der Formel (I): 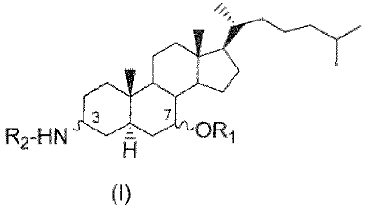 wobei:
R₁ aus H, SO₃H, einer C1-C8-Alkylgruppe, einer C6-C10-Arylgruppe, einer C(=0)R₁₁-Gruppe ausgewählt ist,
R₁₁ aus einer C1-C8-Alkylgruppe, einer C6-C10-Arylgruppe ausgewählt ist,
die Gruppe -NHR₂ ausgewählt ist aus: 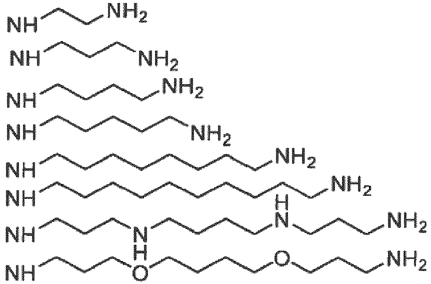 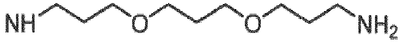 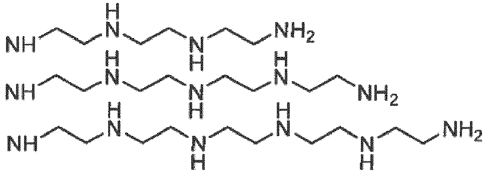 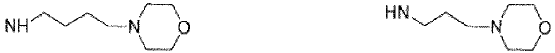 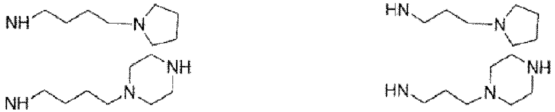 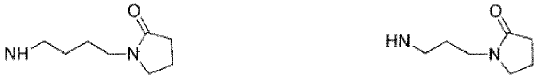 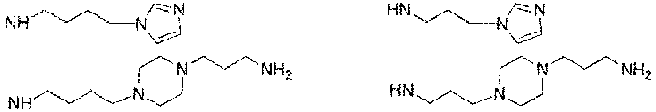 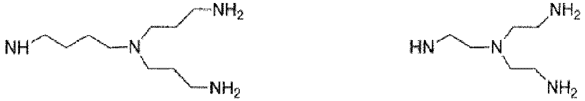
sowie den Stereoisomeren, Gemischen von Stereoisomeren und/oder pharmazeutisch verträglichen Salzen davon zu ihrer Verwendung bei der Behandlung von bakteriellen oder pilzlichen Infektionen bei dem Mensch oder dem Tier.

2. Verbindung der Formel (I) zu ihrer Verwendung nach Anspruch 1, wobei R₁ H ist.

3. Verbindung der Formel (I) zu ihrer Verwendung nach Anspruch 1, ausgewählt aus
- 3β-Spermino-7α-hydroxy-5α-cholestan (SA-1)
- 3β-Spermino-7β-hydroxy-5α-cholestan (SA-2)
- 3β-(1,3-diaminopropan)-7β-hydroxy-5α-cholestan (SA-4)
- 3β-(1,4-diaminobutan)-7β-hydoxy-5α-cholestan (SA-5)
- 3β-(Tris(2aminoethyl)amin)-7β-hydroxy-5α-cholestan (SA-6)
- 3β-(1,5-diaminopentan)-7α-hydroxy-5α-cholestan (SA-7)
- 3β-(1,4-bis(3-aminopropyl)piperazin)-7β-hydroxy-5α-cholestan (SA-8)
- 3β-(1,4-bis-(3-aminopropyl)butan)-7β-hydroxy-5α-cholestan (SA-9)
- 3β-(1-(3-aminopropyl)imidazol)-7α-hydroxy-5α-cholestan (SA-11)
- 3β-(1-(3-Aminopyl)morpholin)-7α-hydroxy-5α-cholestan (SA-12)

4. Verbindung der Formel (I) zu ihrer Verwendung nach einem der vorhergehenden Ansprüche, wobei die Infektion eine bakterielle Infektion ist, insbesondere mit gram + oder gram - Bakterien.

5. Verbindung der Formel (I) zu ihrer Verwendung nach einem der vorhergehenden Ansprüche bei dem Tier, insbesondere bei dem Hund, der Katzen oder den Wiederkäuern.

6. Verbindung der Formel (I) zur Verwendung nach einem der vorhergehenden Ansprüche zur Behandlung von Mastitis, Metritis, Zahninfektionen, Pyoderma oder Otitis bei dem Tier.

7. Verbindung der Formel (I) zur Verwendung nach einem der vorhergehenden Ansprüche, wobei die Verbindung der Formel (I) in Kombination mit einer anderen antibiotischen Verbindung verabreicht wird.

8. Pharmazeutische oder veterinärmedizinische Zusammensetzung, umfassend eine Verbindung der Formel (I) oder ein Stereoisomer, ein Gemisch von Stereoisomeren und/oder ein pharmazeutisch verträgliches Salz einer Verbindung der Formel (I) nach Anspruch 1 und einen pharmazeutisch verträglichen Hilfsstoff.

9. Pharmazeutische oder veterinärmedizinische Zusammensetzung nach Anspruch 8, weiter umfassend eine zweite antibiotische Verbindung.

10. Verbindung der Formel (I) wie in Anspruch 1 definiert.

11. Verfahren zur Herstellung einer Verbindung der Formel (I) nach Anspruch 1, umfassend einen Schritt der reduktiven Aminierung einer Verbindung der Formel (II) in Gegenwart eines Amins R₂NH₂, wodurch die Verbindung der Formel (I) erhalten wird: 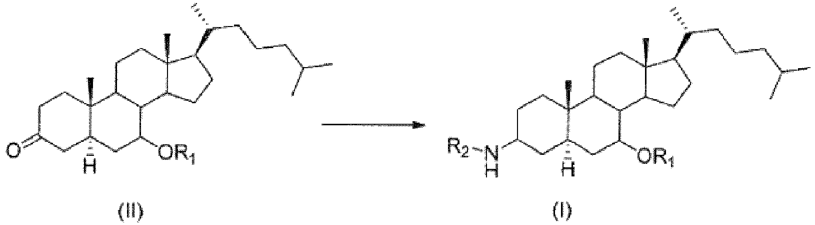 wobei R₁, R₂ wie in Anspruch 1 definiert sind.

## Claims

1. Compound of formula (1): 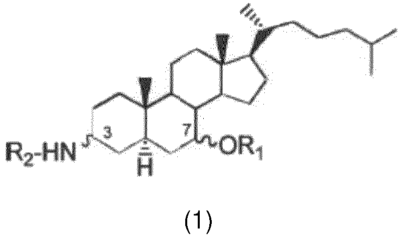 in which:
R₁ is chosen from H, SO₃H, a C₁-C₈ alkyl group, a C₆-C₁₀ aryl group, a C(=O)R₁₁ group,
R₁₁ is chosen from a C₁-C₈ alkyl group, a C₆-C₁₀ aryl group,
the -NHR₂ group is chosen from: 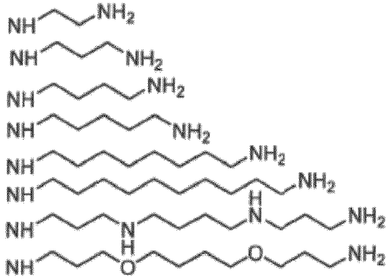 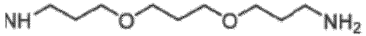 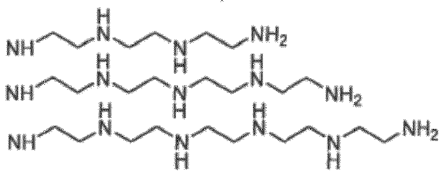 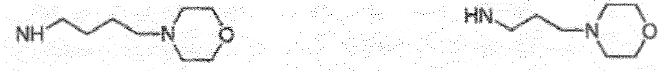 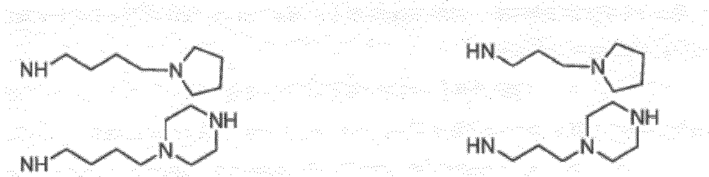 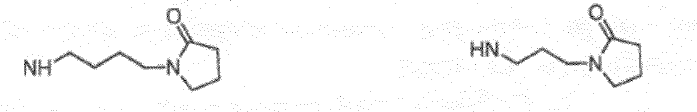 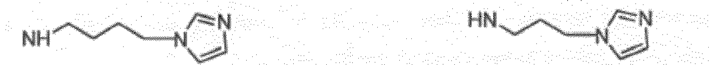 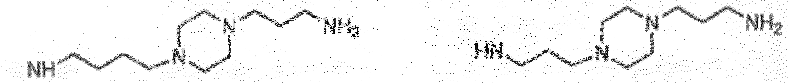 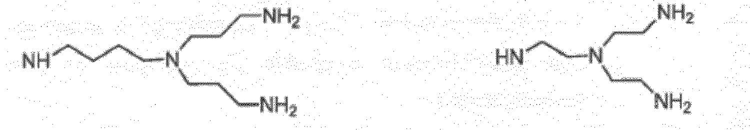
as well as stereoisomers, mixtures of stereoisomers, and/or pharmaceutically acceptable salts thereof, for the use thereof in the treatment of bacterial or fungal infections in humans or animals.

2. Compound of formula (I) for the use thereof according to claim 1, in which R₁ is H.

3. Compound of formula (I) for the use thereof according to claim 1, chosen from:
- 3β-spermino-7α-hydroxy-5α-cholestane (SA-1)
- 3β-spermino-7β-hydroxy-5α-cholestane (SA-2)
- 3β-(1,3-diaminopropane)-7β-hydroxy-5α-cholestane (SA-4)
- 3β-(1,4-diaminobutane)-7β-hydroxy-5α-cholestane (SA-5)
- 3β-(Tris(2aminoethyl)amine)-7β-hydroxy-5α-cholestane (SA-6)
- 3β-(1,5-diaminopentane)-7α-hydroxy-5α-cholestane (SA-7)
- 3β-(1,4-bis(3-aminopropyl)piperazine)-7β-hydroxy-5α-cholestane (SA-8)
- 3β-(1,4-bis-(3-aminopropoxy)butane)-7β-hydroxy-5α-cholestane (SA-9)
- 3β-(1-(3-aminopropyl)imidazole)-7α-hydroxy-5α-cholestane (SA-11)
- 3β-(1-(3-aminopropyl)morpholine)-7α-hydroxy-5α-cholestane (SA-12)

4. Compound of formula (I) for the use thereof according to any preceding claim, in which the infection is a bacterial infection, in particular with gram + or gram - bacteria.

5. Compound of formula (I) for the use thereof according to any preceding claim, in animals, in particular in dogs, cats or rodents.

6. Compound of formula (I) for the use thereof according to any preceding claim, for the treatment of mastitis, metritis, dental infections, pyoderma or otitis in animals.

7. Compound of formula (I) for the use thereof according to any preceding claim, in which the compound of formula (I) is administered in combination with another antibiotic compound.

8. Pharmaceutical or veterinary composition comprising a compound of formula (I) or a stereoisomer, a mixture of stereoisomers and/or a pharmaceutically-acceptable salt of a compound of formula (I) according to claim 1 and a pharmaceutically-acceptable excipient.

9. Pharmaceutical or veterinary composition according to claim 8, further comprising a second antibiotic compound.

10. Compound of formula (I) such as defined in claim 1.

11. Method for preparing a compound of formula (I) according to claim 1, comprising a step of reductive amination of a compound of formula (II) in the presence of an amine R₂NH₂, through which the compound of formula (I) is obtained: 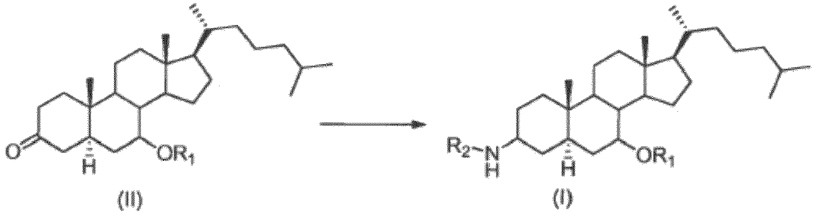 R₁, R₂ being such as defined in claim 1.
